Europäisches Patentamt

European Patent Office

Office européen des brevets

(11) Publication number: **0 080 240 B1**

# EUROPEAN PATENT SPECIFICATION

(45) Date of publication of patent specification: **26.02.86**

(51) Int. Cl.⁴: **C 07 D 201/08, B 01 J 23/58**

(21) Application number: **82201472.6**

(22) Date of filing: **19.11.82**

(54) **Process for the preparation of a lactam.**

(30) Priority: **21.11.81 NL 8105278**

(43) Date of publication of application:
**01.06.83 Bulletin 83/22**

(45) Publication of the grant of the patent:
**26.02.86 Bulletin 86/09**

(84) Designated Contracting States:
**AT BE CH DE FR GB IT LI NL SE**

(56) References cited:
**EP-A-0 017 292**
**LU-A- 53 600**
**US-A-3 235 562**

(73) Proprietor: **STAMICARBON B.V.**
**Postbus 10**
**NL-6160 MC Geleen (NL)**

(72) Inventor: **Van der Stoel, Roland E.**
**Schout Boutenstraat 27**
**NL-6121 HC Buchten (NL)**
Inventor: **Bosma, Marcel A. R.**
**Rembrandtlaan 91**
**NL-6165 AW Geleen (NL)**
Inventor: **Janssen, Petrus H. J.**
**Mauritspark 32**
**NL-6163 HN Geleen (NL)** ·
Inventor: **Van de Moesdijk, Cornelis G. M.**
**Drossaert Saldenstraat 7**
**NL-6181 ER Elsloo (L.) (NL)**

(74) Representative: **Hoogstraten, Willem Cornelis**
**Roeland et al**
**OCTROOIBUREAU DSM Postbus 9**
**NL-6160 MA Geleen (NL)**

Courier Press, Leamington Spa, England.

## Description

The invention relates to a process for the preparation of a lactam by reductive amination, in the gas phase, of a suitable oxoalkane carboxylic acid or of an ester of such an acid with the aid of a hydrogenation catalyst.

According to United States Patent Specification No. 3,235,562, a process of this type can be operated by using a hydrogenation catalyst suitable for gas phase reactions, such as nickel on a carrier such as kieselguhr and copper chromite with barium as promoter.

It has now been discovered that a significantly higher yield can be obtained if the hydrogenation catalyst is promoted with an alkali metal.

The process according to the invention for the preparation of a lactam by reductive amination, in the gas phase of an oxoalkane carboxylic acid with 2 or 3 carbon atoms in the carbon chain between the carbon atom of the carbonyl group and the carbon atom of the carboxyl group, or of an ester of such an acid with the aid of a hydrogenation catalyst containing palladium, platinum or nickel is characterized in that the hydrogenation catalyst is promoted with an alkali metal.

The addition of an alkali metal to the hydrogenation catalyst as a promotor may be carried out in the known way (see, for example, G. H. van den Berg and H. Th. Rijinten, Preparation of Catalysts II, Elseivier, Amsterdam, 1979, page 265) by treating the catalyst with an alkali metal compound such as an alkali metal bicarbonate. The quantity of alkali metal in the catalyst can be varied in a wide range, for example 0.01—5 g of the alkali metal per 100 g of the catalyst (including any carrier material). Preferably 0.1—2 g of the alkali metal is used per 100 g of the catalyst. The alkali metal is preferably sodium.

The reductive amination of the invention may be carried out with known hydrogenation catalysts that contain palladium, platinum or nickel. Catalysts of this type on a carrier material are often used. Carrier materials that are suitable for the process of the invention are, for example, active carbon, graphite, silica, alumina, magnesia, and mixtures of these materials.

The process according to the invention may be carried out at various temperatures, for example temperatures of 50—350°C. The preferred temperature range is 150—250°C.

Like the known reductive amination mentioned above, the reductive amination of the invention is performed by using hydrogen together with ammonia or a primary amine. The quantity of the hydrogen may be varied, for example, between 1 and 25 moles of hydrogen per mole of the starting compound. It is also possible to use more than 25 moles of hydrogen per mole of the starting compound, but there is no advantage in this. The quantity of the ammonia or primary amine may also be varied, for example between 1 and 15 moles per mole of the starting compound. It is possible to use more than 15 moles of the ammonia or primary amine per mole of the starting material, but this does not improve the result. The quantities used per mole of the starting compound are preferably 1—15 moles of hydrogen and 1—5 moles of ammonia or primary amine. If a primary amine is used, the reaction product is an N-substituted lactam with a substituent that corresponds to the primary amine. The primary amine may be any primary amine that will be present in the vapour phase under the reaction conditions. The primary amine or ammonia does not need to be added as such: it may be formed in situ by the reduction of another compound, for example by the reduction of a nitro compound to a primary amine or hydrazine to ammonia.

The starting product may be any of various oxoalkane carboxylic acids or their esters, e.g. 5-oxocaproic acid, 4-methyl-5-oxocaproic acid, levulinic acid or esters of these acids.

The starting product is preferably an ester of the appropriate acid, since a higher yield can be attained with an ester than with the corresponding acid. The ester may be obtained in a known way from the appropriate acid and an alcohol. When using the ester one obtains as by-product the alcohol corresponding to the ester group. Various ester groups may be used, such as an alkyl, cycloalkyl, or benzyl group. Preference is given to the lower alkyl groups, such as a methyl, ethyl, or isopropyl group.

The reductive amination of the invention may be performed by known procedures for gas-phase reactions, for example by passing the gaseous starting mixture through a fixed or fluidized bed of the catalyst. The volumetric flow rate may be varied, for example, between 0.001 and 2 g of the starting compound per millilitre of catalyst material (compacted volume) per hour.

By cooling the resulting gaseous reaction mixture a condensate can be obtained, from which the desired lactam can be separated by distillation or extraction. The lactams obtained are suitable for various applications, for example in the manufacture of plastics.

The invention will now be further explained by means of the following examples.

### Examples I—V

A gaseous mixture of methyl 5-oxocaproate, hydrogen, and ammonia was passed for 165 h down a vertical tubular reactor 25 mm in diameter and 400 mm in length containing a zone of 25 ml (compacted volume) of catalyst and provided with a heating mantle. This gaseous mixture was obtained by evaporating liquid methyl 5-oxocaproate and mixing the vapour with hydrogen and gaseous ammonia. 6 Moles of hydrogen and 3 moles of ammonia were used per mole of the methyl 5-oxocaproate.

The catalyst was palladium on γ-alumina promoted with sodium (Engelhard catalyst with 0.5 wt-% of Pd and 0.4 wt-% of Na). After operating times of 22, 91, 117, 142, and 164 h the conditions were held constant for 1 h and the quantity of methyl 5-oxocaproate that had entered the reactor and the quantity of the reaction product (after condensation at 5°C) were measured. The quantity of methyl 5-oxocaproate that had entered the reactor was determined by measuring the weight loss of the liquid methyl 5-oxocaproate. The composition of the reaction product was determined by gas chromatography. From this determination and the weight of the methyl 5-oxocaproate entering the reactor during the 1 h, the conversion of the oxo-ester and the yield of 6 - methylpiperid - 2 - one can be calculated.

The conversion is understood to mean the quantity of oxo-ester that is converted (i.e. the quantity of oxo-ester that enters the reactor minus the quantity of oxo-ester in the condensed product) expressed as a percentage of the quantity of oxo-ester that enters the reactor. The yield of 6 - methylpiperid - 2 - one is understood to mean the quantity of 6-methylpiperid-2-one in the condensed product expressed as a percentage of the quality of 6 - methylpiperid - 2 - one that theoretically can be formed from the converted quantity of oxo-ester.

The results and the reaction conditions are summarized in the table.

TABLE

| Example | I | II | III | IV | V |
|---|---|---|---|---|---|
| Operating time, in hours | 22 | 91 | 117 | 142 | 164 |
| Temperature, °C | 200 | 200 | 240 | 240 | 240 |
| Space velocity in g oxo-ester per ml catalyst per h | 0.16 | 0.16 | 0.13 | 0.13 | 0.13 |
| Conversion of oxo-ester in % | 98.2 | 68.5 | 92.3 | 85.6 | 79.3 |
| Yield of 6-methylpiperid-2-one | 97.6 | 96.7 | 94.2 | 95.8 | 96.0 |

Comparative example

Example I was repeated without sodium in the catalyst. The yield was 51%.

Example VI

Under the same conditions as the conditions used in Example I, 6 - methylpiperid - 2 - one was prepared with as a catalyst platinum on γ-alumina promoted with sodium (Engelhard catalyst, 0.5 wt-% Pt, 0.25 wt-% Na). The conversion of the oxo-ester was 97% and the yield of 6 - methylpiperid - 2 - one 90%.

**Claims**

1. Process for the preparation of a lactam by reductive amination, in the gas phase, of an oxoalkane carboxylic acid with 2 or 3 carbon atoms in the carbon chain between the carbon atom of the carbonyl group and the carbon atom of the carboxyl group, or of an ester of such an acid with the aid of a hydrogenation catalyst containing palladium, platinum or nickel, characterized in that the hydrogeneration catalyst is promoted with an alkali metal.

2. Process according to Claim 1, characterized in that 0.1—2 g of the alkali metal is used per 100 g of the catalyst.

3. Process according to Claim 1 or 2, characterized in that the alkali metal is sodium.

4. Process according to any of Claims 1—3, characterized in that the reductive amination is performed at a temperature of 150—250°C.

5. Process according to any of Claims 1—4, characterized in that 1—15 moles of hydrogen per mole of the starting compound is used for the reductive amination.

6. Process according to any of Claims 1—5, characterized in that 1—5 moles of ammonia or primary amine per mole of the starting compound is used for the reductive amination.

7. Process according to any of Claims 1—6, characterized in that the starting product is an ester.

8. Process according to Claim 7, characterized in that the ester is the methyl, ethyl, or isopropyl ester.

**Patentansprüche**

1. Verfahren zur Herstellung eines Lactams durch reduktive Aminierung in der Gasphase einer Oxoalkancarbonsäure mit 2 oder 3 Kohlenstoffatomen in der Kohlenstoffkette zwischen dem Kohlenstoffatom der Carbonylgruppe und dem Kohlenstoffatom der Carboxylgruppe, oder eines Esters einer solchen Säure, mit Hilfe eines Hydrierungskatalysators enthaltend Palladium, platin oder Nickel, dadurch gekennzeichnet, daß der Hydrierungskatalysator mit einem Alkalimetal aktiviert ist.

2. Verfahren nach Anspruch 1, dadurch gekennzeichnet, daß 0,1—2 g des Alkalimetalls pro 100 g des Katalysators verwendet werden.

3. Verfahren nach Anspruch 1 oder 2, dadurch gekennzeichnet, daß das Alkalimetall Natrium ist.

4. Verfahren nach einem der Ansprüche 1—3, dadurch gekennzeichnet, daß die reduktive Aminierung bei einer Temperatur von 150—250°C durchgeführt wird.

5. Verfahren nach einem der Ansprüche 1—4, dadurch gekennzeichnet, daß 1—15 Mole Wasserstoff pro Mol der Ausgangsverbindung für die reduktive Aminierung eingesetzt werden.

6. Verfahren nach einem der Ansprüche 1—5, dadurch gekennzeichnet, daß 1—5 Mole Ammoniak oder primäres Amin pro Mol der Ausgangsverbindung für die reduktive Aminierung eingesetzt werden.

7. Verfahren nach einem der Ansprüche 1—6, dadurch gekennzeichnet, daß das Ausgangsprodukt ein Ester ist.

8. Verfahren nach Anspruch 7, dadurch gekennzeichnet, daß der Ester der Methyl-, Äthyl- oder Isopropylester ist.

**Revendications**

1. Procédé de préparation d'un lactame par amination réductrice, en phase gazeuse d'un acide carboxylique oxoalcane avec 2 ou 3 atomes de carbone dans la chaîne carbonée entre l'atome de carbone du groupe carbonyle et l'atome de carbone du groupe carboxyle, ou d'un ester d'un tel acide à l'aide d'un catalyseur d'hydrogénation contenant du palladium, du platine ou du nickel, caractérisé en ce qu'un métal alcalin est promoteur du catalyseur d'hydrogénation.

2. Procédé selon la revendication 1, caractérisé en ce qu'un utilise 0,1—2 g de métal alcalin pour 100 g de catalyseur.

3. Procédé selon la revendication 1 ou 2, caractérisé en ce que le métal alcalin est le sodium.

4. Procédé selon l'une quelconque des revendications 1—3, caractérisé en ce que l'amination réductrice est réalisée à une température de 150—250°C.

5. Procédé selon l'une quelconque des revendications 1 à 4, caractérisé en ce qu'on utilise 1—15 moles d'hydrogène par mole du produit de départ pour l'amination réductrice.

6. Procédé selon l'une quelconque des revendications 1—5, caractérisé en ce qu'on utilise 1—5 moles d'ammoniac ou d'amine primaire par mole du produit de départ pour l'amination réductrice.

7. Procédé selon l'une quelconque des revendications 1—6, caractérisé en ce que le produit de départ est un ester.

8. Procédé selon la revendication 7, caractérisé en ce que l'ester est l'ester méthylique, éthylique ou isopropylique.